# FASCICULE DE BREVET EUROPEEN

(11) **EP 1 778 162 B1**
(45) Date de publication et mention de la délivrance du brevet: **29.06.2016**
(21) Numéro de dépôt: 05796270.6
(22) Date de dépôt: 05.08.2005
(51) Int. Cl.: A61K 36/899, A61Q 17/04, A61Q 19/08, A61K 8/97

(54) **UTILISATION D'UN EXTRAIT DE TRITICUM MONOCOCCUM COMME PRINCIPE ACTIF DANS UNE COMPOSITION COSMETIQUE ET/OU PHARMACEUTIQUE**
VERWENDUNG VON TRITICUM-MONOCOCCUM-EXTRAKT ALS WIRKSTOFF IN EINER KOSMETISCHEN UND/ODER PHARMAZEUTISCHEN ZUSAMMENSETZUNG
USE OF A TRITICUM MONOCOCCUM EXTRACT AS ACTIVE PRINCIPE IN A COSMETIC AND/OR PHARMACEUTICAL COMPOSITION

(30) Priorité: 06.08.2004 FR 0408715
(43) Date de publication de la demande: 02.05.2007
(73) Titulaire: Ashland Specialties France, 06410 Biot (FR)
(72) Inventeur: DAL FARRA, Claude, F-06650 Opio (FR); DOMLOGE, Nouha, F-06560 Valbonne (FR); PEYRONEL, Dominique, F-13014 Marseille (FR)
(74) Mandataire: Macquet, Christophe
(86) Numéro de dépôt international: PCT/FR2005/002033
(87) Numéro de publication internationale: WO 2006/024790

(56) Documents cités:
- WO-A-02/098385
- FR-A- 2 783 418
- ABDEL-AAL, E.-S. M. ET AL: "Compositional and nutritional characteristics of spring einkorn and spelt wheats" CEREAL CHEMISTRY, vol. 72, no. 6, 1995, pages 621-624, XP009044967 ISSN: 0009-0352
- SANCHEZ-MONGE R. ET AL: "Wheat flour peroxidase is a proeminent allergen associated with baker's asthma" CLINICAL AND EXPERIMENTAL ALLERGY, [Online] vol. 27, 1997, pages 1130-1137, XP002320460 Extrait de l'Internet: URL:http://www.blackwell-synergy.com/links /doi/10.1046/j.1365-2222.1997.1340945.x/ab s/> [extrait le 2005-03-08]
- BENAIGES A.: "study of the refirming effect of a plant complex" INTERNATIONAL JOURNAL OF COSMETIC SCIENCE, [Online] vol. 20, no. 4, août 1998 (1998-08), pages 223-233, XP002320461 Extrait de l'Internet: URL:http://www.blackwell-synergy.com/links /doi/10.1046/j.1467-2494.1998.176608.x/abs /> [extrait le 2005-03-08]
- LOJE H ET AL: "Chemical composition,functional properties and sensory profiling of einkorn" JOURNAL OF CEREAL SCIENCE, [Online] vol. 37, 2003, pages 231-240, XP002320489 Extrait de l'Internet: URL:http://www.sciencedirect.com/science?_ ob=MImg&_imagekey=B6WHK-47W568D-C-1&_cdi=6 853&_user=987766&_orig=browse&_coverDate=0 3%2F31%2F2003&_sk=999629997&view=c&wchp=dG LbVtb-zSkzV&md5=0d3a21e540a0734c4d40b5ca4b adf95a&ie=/sdarticle.pdf> [extrait le 2005-03-08]
- E.-S. M. Abdel-Aal ET AL: "Einkorn: A Potential Candidate for Developing High Lutein Wheat", Cereal Chemistry, vol. 79, no. 3, 1 May 2002 (2002-05-01), pages 455-457, XP055063806, ISSN: 0009-0352, DOI: 10.1094/CCHEM.2002.79.3.455
- KASARDA D D ET AL: "N-terminal amino acid sequences of omega-gliadins and omega-secalins", BIOCHIMICA ET BIOPHYSICA ACTA. PROTEIN STRUCTURE AND MOLECULAR ENZYMOLOGY, ELSEVIER, AMSTERDAM; NL, vol. 747, no. 1-2, 14 September 1983 (1983-09-14), pages 138-150, XP025210375, ISSN: 0167-4838, DOI: 10.1016/0167-4838(83)90132-2 [retrieved on 1983-09-14]

## Description

L'invention concerne le domaine de la cosmétique ainsi que le domaine de la pharmaceutique, notamment le domaine de la dermatologie.

La présente invention a pour objet l'utilisation, à titre de principe actif, dans ou pour la préparation d'une composition cosmétique et/ou dermatologique et/ou pharmaceutique, d'une quantité efficace d'un extrait d'une espèce végétale diploïde appartenant au genre Triticum. Ledit extrait ou la composition le contenant étant, entre autres, destinés à prévenir ou traiter les dommages cellulaires provoqués par les radicaux libres induits, notamment, par les polluants atmosphériques et/ou par le rayonnement ultraviolet.

Le vieillissement des organismes et des organes, tels que la peau, est un processus évident mais aussi extrêmement complexe qui peut prendre des formes variées d'un individu à un autre. Le vieillissement de la peau n'est qu'un seul aspect du vieillissement global de l'organisme. Divers facteurs interviennent dans ce processus, des facteurs d'origines endogènes bien sûr, mais aussi des facteurs d'origines exogènes. Ainsi, une exposition excessive aux rayonnements solaires et, notamment, aux rayons ultraviolets, est un phénomène qui accélère le vieillissement, on parle d'ailleurs d'un vieillissement photo-induit. On assiste ainsi à un déclin prématuré de la quantité et de la qualité des constituants de la peau et de l'épiderme en particulier. En effet, l'action de la lumière et, notamment, des rayonnements UV entraînent la formation de radicaux libres. D'autres facteurs d'origine extérieure, tels que la pollution et les agressions de toutes sortes peuvent agir de façon néfaste sur la peau. On sait, par exemple, que la toxicité des polluants atmosphériques, notamment des polluants gazeux tels que le dioxyde de souffre, l'ozone et les oxydes d'azote, est liée en grande partie à leur pouvoir initiateur de radicaux libres. Les cellules de la peau, en contact direct avec le milieu extérieur, sont donc particulièrement exposées à ces polluants.

Les radicaux libres sont des sources de phénomènes d'oxydation. Ce sont des constituants chimiques très réactionnels qui interviennent de manière transitoire dans de nombreux mécanismes cellulaires. Les radicaux libres photo-induits proviennent en grande partie de l'oxygène moléculaire. Etant donné son abondance dans l'organisme et sa capacité à accepter les électrons, les radicaux libres qui en dérivent sont les plus nombreux à participer aux réactions radicalaires. On peut ainsi dénombrer parmi ces espèces réactives : l'oxygène singulet (produit de l'excitation de l'oxygène moléculaire par les photons) ; le radical anion superoxyde (produit de l'addition d'un électron sur l'oxygène moléculaire) ; le peroxyde d'hydrogène (non radicalaire mais qui peut donner lieu à la production de radicaux hydroxyles) ; le radical hydroxyle (très oxydant donc très réactif et très toxique pour les cellules).

Ces radicaux libres, s'ils sont incontrôlés, peuvent rapidement réagir avec des molécules avoisinantes donnant naissance à des produits toxiques pouvant interférer avec le fonctionnement physiologique normal des cellules. Par les phénomènes d'oxydation et les attaques radicalaires qu'ils engendrent, les radicaux libres vont produire un stress oxydant et provoquer des dégâts importants. Ils vont, par exemple, provoquer des dommages au niveau des membranes cellulaires ; ils vont altérer des macromolécules (péroxydation lipidique, carbonylation des protéines) et engendrer des mutations et des ruptures au niveau des brins d'ADN, ce qui peut conduire à la mort de la cellule. Ils sont ainsi souvent associés aux phénomènes de nécrose des tissus. La présence de ces radicaux libres dans la peau est probablement responsable d'un grand nombre d'effets indésirables. Par exemple, le vieillissement est observé prématurément au niveau de la peau comme une conséquence du photo-vieillissement, accélérant entre autres les phénomènes de détérioration de l'élastine, du collagène ou de la fibronectine. Ces dommages peuvent parfois aboutir à des processus de cancérisation. Il est donc important de mettre au point des systèmes capables de lutter activement contre ces réactifs et leurs conséquences.

De plus, ces radicaux libres sont des substances pro-inflammatoires, c'est-à-dire qu'elles favorisent le déclenchement de réactions inflammatoires de la peau, (Briganti S at Al., JEADV (2003) 17, 663-669) en agissant, notamment, comme second messager dans l'induction de différentes réponses cellulaires.

L'organisme possède des mécanismes enzymatiques de défense contre ces radicaux libres, trois enzymes constituent les clés de voûte de cette protection : la famille des superoxydes dismutases (SOD) qui dismutent l'ion superoxyde en peroxyde d'hydrogène; les catalases, généralement confinées dans les peroxisomes, accélérant la réaction spontanée qui transforme le peroxyde d'hydrogène en oxygène et en eau ; et la glutathion peroxydase, enzyme cytosolique séléniée réduisant le peroxyde d'hydrogène en présence de glutathion. Cependant ces systèmes de défenses antioxydantes s'avèrent souvent insuffisants devant les nombreux stress et agressions extérieures auxquels sont soumis les organismes et la peau en particulier.

De nombreux actifs ont déjà été mis au point afin de combattre activement ces radicaux libres et les réactions d'oxydation qu'ils engendrent. On peut notamment citer, par exemple, les vitamines A, C ou E, les polyphénols (notamment ceux d'origine végétale), ou encore la quinoléine et ses dérivés. Cependant, ces actifs ne permettent pas de résoudre ces problèmes de manière réellement satisfaisante, et de nombreux progrès restent encore à faire afin de pouvoir disposer d'actifs présentant des propriétés véritablement convenables. De nombreux acides aminés ont aussi été utilisés en tant qu'agent antioxydant. Cependant, ils présentent le désavantage d'être très rapidement métabolisés dans les cellules et possèdent ainsi une très faible efficacité. Il subsiste donc toujours un besoin d'actif ayant une action réellement efficace en tant qu'agent antioxydant et/ou en tant qu'agent anti-radicalaire.

Pour ce qui concerne l'état de la technique de l'invention, on notera en particulier les documents suivants : WO02/098385 divulguant des préparations cosmétiques contenant une quantité efficace d'un extrait de plantes germantes, Loje et al., « Chemical composition, functional properties and sensory profiling of einkorn », Journal of Cereal Science, vol.7, 2003, Abdel-Aal et al., « Einkorn : a potential candidate for developing high lutein wheat », Cereal Chemistry, vol. 79, No 3, 2002, et Kasarda et al., « N-terminal amino acid sequences of omega-gliadins and omega-secalins », Biochimica et biophysica acta, Protein structure and molecular enzymology, vol. 747, no 1-2, p. 138-150, 1983.

La présente invention vise à combler cette lacune et a pour principal objectif de fournir un nouveau principe actif antioxydant et/ou anti-radicalaire. Les inventeurs ont réussi à sélectionner des substances particulières présentant des propriétés remarquables lorsque celles-ci sont appliquées sur la peau. Ils ont ainsi découvert, de manière inattendue, qu'un extrait d'une espèce végétale diploïde appartenant au genre Triticum possède des propriétés antioxydantes lorsque celui-ci est appliqué sur la peau.

Ainsi, selon un premier aspect, la présente invention a pour objet une composition comprenant une quantité efficace d'au moins un extrait aqueux de *Triticum monococcum,* dans un milieu cosmétiquement acceptable, destinée à être utilisée comme agent photo-protecteur dans le traitement des agressions dues aux rayonnements UV.

Selon un autre aspect, la présente invention a pour objet une composition comprenant une quantité efficace d'un extrait aqueux de graines de *Triticum monococcum,* dans un milieu cosmétiquement acceptable, seul ou en association avec au moins un autre agent actif, destinée à être utilisée en tant qu'agent antioxydant et/ou anti- radicalaire, dans le traitement du stress oxydatif.

Selon un autre aspect encore, la présente invention a pour objet une composition comprenant une quantité efficace d'un extrait aqueux de graines de *Triticum monococcum,* dans un milieu cosmétiquement acceptable, destinée à être utilisée dans le traitement de la peau et les phanères contre tous les types d'agressions extérieures.

Selon un autre aspect enfin, la présente invention a pour objet une composition dermatologique, comprenant dans un milieu pharmaceutiquement acceptable, au moins une quantité efficace d'un extrait aqueux de graines de *Triticum monococcum,* destinée à être utilisée dans le traitement de l'inflammation cutanée.

Le *Triticum monococcum* communément est appelé engrain ou petit épeautre.

A la connaissance de la demanderesse, il n'a jamais été proposé, dans des documents antérieurs, des compositions cosmétiques contenant au moins un extrait d'une espèce végétale diploïde, appartenant au genre Triticum.

Les végétaux du genre triticum, appartenant à la famille des Graminées (Poaceae) détermine les espèces céréalières auxquelles il est légitime de donner le nom de blé. Le blé est la céréale la plus cultivée et la plus consommée aujourd'hui dans le monde. Domestiqué au Proche-Orient à partir d'une graminée sauvage il y a environ 10 000 ans, il compte actuellement quelque 30 000 formes cultivées. C'est une céréale dont le grain est un fruit sec et indéhiscent, appelé caryopse, constitué d'une graine et de téguments. Les blés sont des plantes herbacées annuelles, monocotylédones, à feuilles alternes, formées d'un chaume portant un épi constitué de deux rangées d'épillets sessiles et aplatis. Les fleurs sont dépourvues de pétales et entourées de pièces écailleuses (glumelles). On compte plus d'une quinzaine d'espèces de céréales proches parentes du blé commun. Les deux espèces les plus cultivées sont le blé tendre ou blé commun (*Triticum aestivum*) et le blé dur (*Triticum durum*) mais il existe de nombreuses autres espèces de Triticum qui se différencient par leur degré de ploïdie et par leur nombre de chromosomes.

Trois groupes de Triticum sont connus et ont été utilisés par l'homme pour son alimentation. Ils sont répartis selon leur niveau de ploïdie :
- Le groupe diploïde (2 × 7 chromosomes) comprend *Triticum monococcum* (engrain ou petit épeautre) et *T. spontaneum,* qui font partie des formes les plus anciennement cultivées, caractérisées par des épis grêles où les grains restent enveloppés par les glumelles ;
- Le groupe tétraploïde (4 × 7 chromosomes) comprend *T. dicoccoides* (amidonnier sauvage), *T. dicoccum* (amidonnier), *T. turgidum* et *T. durum* (blé dur), à épis denses dont les graines riches en gluten servent à fabriquer les pâtes alimentaires ;
- Le groupe hexaploïde (6 × 7 chromosomes), représenté par *T. vulgare,* ou *T. aestivum* (blé tendre) et *T. spelta* (épeautre), comprend la majorité des blés à épis assez larges et aux graines riches en amidon nécessaires à la fabrication du pain.

L'engrain (Triticum monococcum), appelé parfois « petit épeautre » est un blé diploïde, très ancien, dont les premières traces de culture datent d'environ 9000 ans avant JC (Toussaint-Samat, 1992). C'est un blé primitif, véritable ancêtre des céréales modernes polyploïdes. C'est une plante bien adaptée aux sols chauds et secs, pauvres, pierreux et sableux des zones montagneuses d'Europe de l'Ouest. C'est une espèce de petite taille à très longue saison végétative et à très faibles rendements. Les plants ont une paille et un épi courts ainsi que de petits grains. Aujourd'hui, l'engrain est cultivé de façon marginale dans le pourtour méditerranéen (Kimber et Feldman, 1987). Sa culture est très limitée, il est utilisé essentiellement pour ses propriétés agronomiques, notamment, afin d'aider à l'amélioration des blés modernes. Il possède, en effet, de grandes qualités agronomiques : par comparaison au blé dit « commun », l'engrain est généralement plus résistant aux maladies et possède la capacité de résister à la sécheresse ainsi qu'à une certaine salinité (Sharma et al., 1981). Par ailleurs, des études récentes ont mis en évidence des qualités nutritionnelles remarquables des blés diploïdes : ils sont considérés comme plus nutritif que les blés « communs ». En effet, ils possèdent notamment un taux particulièrement élevé en protéines (Vallega 1992) mais aussi en caroténoïdes, en phosphore et en potassium (Abdel-Aal et al., 1995 ; Mtuz et al., 2000). En outre, un des plus grand intérêts des blés diploïdes provient du fait que les protéines de *T. monococcum,* notamment, semblent être non toxiques vis-à-vis des individus souffrant de maladies coeliaques (Auricchio et al. 1982) et qu'il n'est pas un facteur induisant ce type de maladie (Favret et al. 1984, 1987). Ainsi, ces avantages nutritionnels et, notamment, sa consommation possible par les individus allergiques au blé commun, donnent à l'engrain et aux autres blés diploïdes un nouvel intérêt.

Selon une méthode de réalisation actuellement préférée de l'invention, l'extrait végétal, appartenant au genre Triticum, est un extrait de nature aqueuse.

Plus précisément l'extrait selon l'invention contient des composés de faible poids moléculaire. Il contient ainsi, entre autres, des sucres et des composés de nature peptidique. Parmi cette fraction de composés de nature peptidique, l'extrait selon l'invention contient une quantité importante d'acides aminés soufrés, il contient notamment beaucoup d'acides aminés cystéine et des résidus de glutathion. Par extrait de nature peptidique on entend tous composés de nature protéique, tels que les fragments de protéines, les peptides mais aussi les acides aminés libres. Les peptides, acides aminés et fragments de protéines sont dosés selon les techniques classiques, bien connues de l'homme du métier.

L'extrait de plantes appartenant à une espèce végétale diploïde du genre Triticum, doit s'entendre comme un extrait d'au moins un végétal diploïde appartenant au genre Triticum. Selon un mode de réalisation avantageux de l'invention, la plante utilisée afin d'obtenir l'extrait selon l'invention est le Triticum monococcum communément appelé engrain ou petit épeautre. Préférentiellement, l'extrait selon l'invention est obtenu à partir de la graine de ces végétaux. Ainsi, selon une méthode de réalisation préférée de l'invention, l'extrait aqueux selon l'invention est obtenu à partir des graines de Triticum monococcum. L'extrait est aussi avantageusement réalisé à partir de farines issues de ces graines.

Toute méthode d'extraction ou de purification connue de l'homme du métier peut être utilisée afin de préparer l'extrait selon l'invention. Les étapes d'extraction selon l'invention seront avantageusement réalisées à partir de farine, tout type de farines pouvant être utilisé. Cet extrait est mis en solution dans un ou plusieurs solvants. On peut en particulier citer des solvants aqueux. Par solvant aqueux, on entend tout solvant constitué totalement ou partiellement d'eau. On peut citer l'eau elle-même, les solvants hydroalcooliques en toutes proportions ou encore les solvants constitués d'eau et d'un composé comme le propylène glycol ou le butylène glycol en toutes proportions.

Un aspect essentiel de l'invention est l'utilisation d' un extrait tel que défini précédemment, dans ou pour la préparation d'une composition cosmétique et/ou pharmaceutique, à usage topique, destinée, notamment, à obtenir une activité protectrice vis-à-vis des espèces réactives de l'oxygène. Lesdits extraits étant avantageusement utilisés en tant qu'agent antioxydant et/ou en tant qu'agent anti-radicalaire. Par agent anti-radicalaire, on entend tout composé capable de piéger les radicaux libres. Cet actif est, en effet, capable de bloquer les réactions en chaînes des radicaux libres avant les étapes ultimes de dégradation des constituants biologiques de la peau, on parle alors de composés antioxydants.

L'extrait selon l'invention peut être avantageusement utilisé dans ou pour la préparation d'une composition cosmétique et/ou pharmaceutique, en tant qu'agent photo-protecteur et, plus particulièrement, en tant qu'agent photo-protecteur dit « secondaire ». On distingue, en effet, les agents photo-protecteurs primaires des agents photo-protecteurs secondaires. Les agents photo-protecteurs primaires sont des substances qui exercent un pouvoir physique : ils sont en mesure d'absorber les rayonnements UV et de les restituer sous forme de chaleur afin de protéger la peau. Les agents photo-protecteurs secondaires sont des substances qui ont plutôt un effet biologique ; ce sont, par exemple, les agents de type antioxydant qui interrompent les chaînes de réactions photo-chimiques qui sont déclenchées lorsque le rayonnement UV pénètre dans la peau.

Par ses activités particulières, les extraits selon l'invention pourront être utilisés avantageusement dans ou pour la préparation d'une composition cosmétique et/ou pharmaceutique, à usage topique, destinée, notamment, à lutter de manière préventive et/ou curative contre les manifestations du vieillissement cutané et, tout particulièrement, afin de lutter contre et/ou de prévenir le vieillissement photo-induit. Par manifestations cutanées du vieillissement on entend toutes modifications de l'aspect extérieur de la peau dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, mais également toutes modifications internes de la peau qui ne se traduisent pas systématiquement par un aspect extérieur modifié comme, par exemple, toutes dégradations internes de la peau consécutives à une exposition aux rayonnements ultra-violets. L'extrait selon l'invention ou la composition le contenant permettra de lutter, en particulier, contre la perte d'élasticité et de fermeté de la peau.

L'extrait selon l'invention permet de protéger la peau contre tous types d'agressions extérieures. Ainsi il peut être destiné à protéger les substrats kératiniques et, plus particulièrement, à protéger la peau et/ou les phanères contre tous les types d'agressions extérieures. L'utilisation de l'extrait, ou d'une composition le contenant, va permettre aux substrats kératiniques d'être protégés et de mieux résister au stress que produit l'environnement.

On entend, par le terme "agression extérieure", les agressions que peut produire l'environnement. A titre d'exemple, on peut citer des agressions telles que la pollution, les UV, ou encore les produits à caractère irritant tels que les tensioactifs, les conservateurs ou les parfums. Par pollution, on entend aussi bien la pollution « extérieure », due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldehydes), ou bien encore la fumée de cigarette.

L'extrait selon l'invention permettra tout particulièrement de protéger la peau et/ou les phanères des agressions qui aboutiraient à la formation de radicaux libres et créeraient ainsi un stress oxydatif. Ainsi, l'extrait selon l'invention permettra de lutter contre les dommages esthétiques provoqués sur la peau et/ou les cheveux par les radicaux libres induits, notamment, par les polluants atmosphériques et par le rayonnement. L'extrait pourra être utilisé dans ou pour la préparation d'une composition cosmétique et/ou pharmaceutique destinée à prévenir ou traiter les dommages cellulaires provoqués par les radicaux libres induits, entre autres, par les polluants atmosphériques et/ou par le rayonnement ultraviolet. Plus généralement, par son action antioxydante, l'extrait selon l'invention pourra être utilisé dans ou pour la fabrication d'une composition destinée à protéger la peau contre le stress, notamment contre le stress oxydatif.

Par ailleurs, selon un autre aspect, l'extrait selon l'invention, ou la composition le contenant, permettra avantageusement de lutter contre les manifestations de l'inflammation résultant de ces agressions ; il permettra notamment de lutter contre les manifestations cutanées de l'inflammation.

Selon un autre aspect, l'invention concerne une composition cosmétique et/ou dermatologique caractérisée en ce qu'elle contient, dans un milieu acceptable, comme principe actif, au moins un extrait tel que défini précédemment. Selon une méthode de réalisation particulièrement avantageuse de l'invention, la composition contient un extrait Il est bien entendu que l'extrait selon l'invention peut être utilisé seul ou en association avec au moins un autre agent actif.

La composition contenant l'extrait selon l'invention peut être une composition cosmétique ou dermatologique ou pharmaceutique. Préférentiellement selon l'invention, la composition est une composition cosmétique car elle est destinée à améliorer l'aspect et les performances cutanées générales de l'individu qui en fait usage. La composition selon l'invention est préférentiellement une composition cosmétique et/ou dermatologique adaptée à l'administration par voie topique cutanée comprenant un milieu cosmétiquement ou pharmaceutiquement acceptable. L'invention concerne, en particulier, une composition cosmétique destinée à obtenir une action antioxydante et/ou une action anti-radicalaire lorsqu'elle est appliquée sur la peau.

Il est bien évident que l'invention s'adresse aux mammifères en général et plus particulièrement aux êtres humains. Selon un mode de réalisation avantageux de l'invention, les extraits précités selon l'invention sont, préalablement à leur utilisation, solubilisés dans un ou plusieurs solvants cosmétiquement ou pharmaceutiquement acceptables, classiquement utilisés par l'homme du métier, comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants. Selon encore un autre mode de réalisation avantageux de l'invention, les extraits précités sont, préalablement à leur utilisation, solubilisés dans un vecteur cosmétique ou pharmaceutique comme les liposomes ou adsorbés sur des polymères organiques poudreux, des supports minéraux comme les talcs et bentonites, et plus généralement solubilisés dans, ou fixés sur, tout vecteur cosmétiquement ou pharmaceutiquement acceptable.

Il est bien entendu évident que l'extrait selon l'invention peut être utilisé seul ou bien en association avec au moins un autre agent actif, dans ou pour la préparation d'une composition cosmétique et/ou dermatologique et/ou pharmaceutique. La quantité efficace de principe actif correspond à la quantité nécessaire afin d'obtenir le résultat désiré. Selon un mode de réalisation avantageux de l'invention, l'extrait est présent, dans la composition le contenant, à une quantité comprise entre 0,0001 % et 10 % en poids, préférentiellement à une quantité comprise entre 0,001 % et 5 % en poids, et d'une manière encore plus préférentielle à une quantité comprise entre 0,05 % et 3 % en poids par rapport au poids total de la composition finale.

Quelle que soit la forme de l'invention, la composition selon l'invention peut être ingérée, injectée ou appliquée sur la peau (sur toute zone cutanée du corps), les cheveux, les ongles ou les muqueuses. Selon le mode d'administration, la composition selon l'invention peut se présenter sous toutes les formes galéniques normalement utilisées. Préférentiellement, les compositions selon la présente invention se présenteront sous une forme galénique adaptée à l'administration par voie topique cutanée. Elles couvrent toutes les formes cosmétiques ou dermatologiques. Ces compositions doivent donc contenir un milieu cosmétiquement acceptable, c'est-à-dire compatible avec la peau, les poils ou les cheveux.

Ces compositions peuvent notamment se présenter sous forme d'une solution aqueuse, hydroalcoolique ou huileuse ; d'une émulsion huile-dans-eau, eau-dans-huile ou émulsions multiples ; elles peuvent aussi se présenter sous forme de crèmes, de suspensions ou encore de poudres, adaptées à une application sur la peau, les muqueuses, les lèvres et/ou les cheveux. Ces compositions peuvent être plus ou moins fluides et avoir l'aspect d'une crème, d'une lotion, d'un lait, d'un sérum, d'une pommade, d'un shampooing, d'un gel, d'une pâte ou d'une mousse. Elles peuvent aussi se présenter sous forme solide, comme un stick ou être appliquées sur la peau sous forme d'aérosol. Elles peuvent être utilisées comme produit de soin et/ou comme produit de maquillage de la peau. Pour l'injection, la composition selon l'invention peut se présenter sous forme de lotion aqueuse, huileuse ou sous forme de sérum. Pour l'application sur les yeux, la composition peut se présenter sous forme de gouttes et pour l'ingestion, elle peut se présenter sous forme de capsules, de granulés, de sirops ou de comprimés. Ces compositions comprennent, en outre, tout additif usuellement utilisé dans le domaine d'application envisagé ainsi que les adjuvants nécessaires à leur formulation, tels que des solvants, des épaississants, des diluants, des antioxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc. Dans tous les cas, l'homme du métier veillera à ce que ces adjuvants ainsi que leurs proportions soient choisis de telle manière à ne pas nuire aux propriétés avantageuses recherchées de la composition selon l'invention. Ces adjuvants peuvent, par exemple, correspondre de 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition. Bien entendu, l'homme du métier veillera à choisir les éventuels composés complémentaires, actifs ou non-actifs, et/ou leurs quantités, de telle sorte que les propriétés avantageuses du mélange ne soient pas, ou sensiblement pas, altérées par l'adjonction envisagée.

Les compositions selon l'invention trouvent une application notamment comme compositions cosmétiques ou pharmaceutiques pour la peau, les muqueuses et/ou les semi-muqueuses, mais aussi comme composition cosmétique pour les cheveux et/ou les poils. Elles trouvent une application toute particulière en tant que produit de protection et/ou de soin de la peau. La composition selon l'invention est, préférentiellement, une composition capable d'entretenir ou de soigner la peau, mais c'est aussi une composition apte à prévenir et/ou à lutter contre les manifestations du vieillissement cutané, particulièrement contre le vieillissement photo-induit. La composition selon l'invention est, de la même manière, capable de protéger la peau contre les agressions extérieures provoquées, notamment, par l'action du rayonnement solaire ou par d'autres agents physiques, chimiques ou biologiques, en agissant principalement par suppression de la formation de radicaux libres oxygénés et des réactions qu'ils entraînent.

On peut également envisager une application dans le domaine des compositions de maquillage de la peau du visage et du corps, tels que les rouges à lèvres, les fonds de teint, les crèmes teintées, les sticks anti-cernes, les compositions anti-solaires ou de bronzage artificiel. Les compositions, objet de l'invention, trouvent leur application dans grand nombre de traitements notamment cosmétiques ou dermatologiques, et elles peuvent constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps. La composition selon l'invention peut également consister en des préparations solides comprenant également des savons ou des pains de nettoyage. La composition peut être aussi conditionnée sous forme d'une composition pour aérosol comprenant également un agent propulseur sous pression. La composition peut être aussi à usage bucco-dentaire, comme par exemple une pâte de dentifrice. La composition de l'invention peut aussi être une composition cosmétique destinée à une administration par voie orale. Pour une administration par voie orale, la composition selon l'invention peut se présenter sous toutes formes adaptées, particulièrement sous forme d'une solution buvable, d'un sirop, d'un comprimé, d'une dragée, d'une gélule ou encore d'un aliment ou complément nutritionnel.

Selon un autre aspect, la présente invention concerne un procédé de traitement cosmétique pour traiter les peaux, notamment les peaux âgées et/ou pour combattre les phénomènes de vieillissement cellulaire consistant à appliquer sur la surface de la peau une quantité efficace de la composition telle que définie précédemment afin d'obtenir l'action désirée. Selon une méthode de réalisation préférée de l'invention, le procédé selon l'invention permet de lutter contre le vieillissement photo-induit.

La présente invention concerne, de la même manière, un procédé de traitement cosmétique afin de protéger la peau et/ou les phanères contre tous types d'agressions extérieures. La présente invention concerne, de la même manière, un procédé de traitement cosmétique permettant de lutter contre les dommages inesthétiques provoqués sur la peau et les cheveux par les radicaux libres induits notamment par les polluants atmosphériques et le rayonnement ultraviolet. Ce procédé consistant à appliquer sur la peau ou les cheveux une composition telle que définie précédemment. La présente invention est aussi relative à un procédé de traitement de l'inflammation cutanée consistant à appliquer sur la peau ou les cheveux une composition telle que définie précédemment.

Des modes de réalisation particuliers de ce procédé de traitement cosmétique résultent également de la description précédente. Le procédé de traitement cosmétique de l'invention peut être mis en oeuvre notamment en appliquant les compositions cosmétiques telles que définies ci-dessus, selon la technique d'utilisation habituelle de ces compositions, par exemple : application de crèmes, de gels, de sérums, de lotions, de laits, de shampooings ou de compositions anti-solaires, sur la peau ou sur les cheveux, ou encore, application de dentifrice sur les gencives.

D'autres avantages et caractéristiques de l'invention apparaîtront mieux à la lecture des exemples donnés à titre illustratif et non limitatif.

### Exemple 1 : Préparation d'un extrait d'une espèce végétale diploïde appartenant au genre Triticum

La préparation de cet extrait est effectuée à partir de tout genre de farine, préférentiellement de la farine issue de l'espèce triticum monococcum sera utilisée, des farines raffinées pourront être utilisées. Selon une méthode de réalisation de l'invention préférée, on utilisera une farine complète.

L'extraction des matières actives de la farine peut être réalisée selon différentes manières. Une des principaux modes d'extraction sera une extraction par voie aqueuse.

Une quantité de farine (1 kg) est mise en suspension dans une solution aqueuse (dilution au 1/10). Ce mélange est maintenu sous agitation pendant un temps suffisamment long permettant la solubilisation des fractions solubles, mais permettant d'éviter l'obtention d'un gonflement trop important qui occasionnerait des pertes par rétention d'eau. La température d'extraction est variable (comprise entre 4 et 80°C) ; préférentiellement l'opération sera réalisée à froid.

Après cette phase d'extraction le milieu est centrifugé ou essoré, puis filtré sur filtre-plaque de porosité de plus en plus réduite jusqu'à obtention d'un liquide brillant. L'opération est réalisée deux à trois fois. Ce filtrat est ensuite soumis à une opération permettant la séparation des molécules de faible poids moléculaire par rapport aux molécules de fort poids moléculaire (tel que l'amidon ou les protéines). Cette opération est réalisée par précipitation des molécules de fort poids moléculaire par l'ajout d'alcool ou de sels. Préférentiellement une filtration tangentielle sera réalisée. Au cours de cette opération le retentât est écarté et le filtrat (dialysât) est conservé. Une étape de concentration de ce filtrat sera ultérieurement effectuée.

Au cours de cette étape d'extraction diverses opérations peuvent être effectuées afin de favoriser l'extraction des fractions peptidiques désirées. A cette fin, l'adjonction d'enzymes est particulièrement indiquée, ces enzymes seront choisies parmi les enzymes ayant une action sur l'amidon, la cellulose et/ou les protéines.

Selon une variante à ce procédé, l'extraction des matières actives de la farine d'épeautre pourra être réalisée en milieu hydroalcoolique ou en milieu hydroglycolique.

A la fin de ce procédé d'extraction des principes actifs obtenu à partir d'une espèce végétale diploïde appartenant au genre Triticum, l'on obtiendra une solution titrant à environ 5 g/L en matière azoté.

### Exemple 2 : Mise en évidence de l'effet anti-oxydant de l'extrait selon l'invention sur des fibroblastes.

### I. Protocole expérimental :

Des fibroblastes primaires, à un passage compris entre P6 et P13, ont été ensemencés sur des boîtes de diamètre 100 à environ 250 000 cellules par boîte. Ces cellules sont ensuite incubées à 37°C, dans une atmosphère saturée à 5 % de CO₂, jusqu'à ce qu'elles atteignent 60 à 70 % de confluence.

Une fois la confluence désirée atteinte, les cellules reçoivent l'actif, ou du milieu de culture, pendant une durée de 24 heures. Ces cellules sont ensuite irradiées, ou non, par des UVB à 100 mJ/cm². Pendant le temps d'irradiation, les boîtes sont traitées avec du PBS seul ou avec le produit à tester dilué dans du PBS.

Quatre conditions d'étude ont ainsi été réalisées : deux conditions contrôles avec du milieu de culture et avec, ou sans, irradiation aux UVB ; deux conditions avec l'extrait de l'exemple 1 (dilué à 1 % dans du milieu de culture) et avec, ou sans, irradiation aux UVB.

Les cellules sont traitées différemment suivant les tests effectués ultérieurement :
- Dans le cas du dosage de l'activité catalase, les cellules sont remises en culture pendant 30 minutes avec ou sans la présence de l'actif selon l'invention.
- Pour le dosage de la carbonylation des protéines, les cellules sont directement arrêtées après l'irradiation.
- Pour la révélation de l'activité SOD, les cellules sont remises en culture pendant 24 heures, en présence ou non, de l'actif selon l'invention.

Les échantillons à tester sont préparés en récupérant toutes les cellules des diamètres 100 et en les lysant avec un tampon adéquat contenant des inhibiteurs de protéases ainsi qu'en les « scrappant ». La suspension cellulaire obtenue est alors broyée (par passage à la seringue) afin d'extraire le plus de protéines possibles. Puis, finalement, les extraits protéiques sont centrifugés à 12500 rpm durant 5 min pour ne récupérer que le surnageant. Un dosage de protéines par BCA est effectué afin de standardiser tous les échantillons à la même concentration.

### II. Mise en évidence de l'activité de la Super Oxyde Dismutase :

La Super Oxyde Dismutase (SOD) est un système enzymatique qui assure une protection efficace contre les radicaux libres. Cette enzyme catalyse en effet la dismutation d'un radical libre, l'anion superoxyde, en peroxyde d'hydrogène. Ce peroxyde d'hydrogène sera éliminé par la catalase.

Le principe de ce test est basé sur une migration des protéines totales d'un extrait cellulaire, sur un gel de polyacrylamide (gel page natif), qui sera ensuite coloré afin de permettre la mise en évidence de l'activité de la SOD.

Les échantillons à tester, correspondant aux quatre conditions décrites précédemment, sont déposés sur un gel de polyacrylamide à 8 % et soumis à une électrophorèse durant 2 heures à 100 V. A la fin de la migration, le gel est coloré par un mélange de NBT (Nitrobleu de tetrazolium) et de riboflavine. Pour ce test, les échantillons ont reçu une application de l'actif selon l'invention à 1 % pendant l'irradiation aux UVB à 100 mJ/cm² et 24 heures avant et après le stress.

L'absence de coloration démontre la présence d'une activité SOD. La riboflavine, en présence de lumière, forme un anion superoxyde pris en charge par le NBT et résultant en une coloration du gel en violet. En présence de la SOD, l'anion superoxyde sera dégradé, et la coloration violette ne se fera donc pas. Les bandes achromatiques obtenues peuvent ensuite être quantifiées par rapport à leur intensité.

Le tableau ci-dessous illustre le résultat de la quantification de l'intensité des bandes, résultant de l'activité de la SOD, en fonction des différentes conditions d'étude.

| **Conditions d'étude** | **% d'intensité des bandes** |
|---|---|
| - actif / - UVB | 100 |
| - actif / + UVB | 146,15 |
| + actif / - UVB | 123,08 |
| + actif / + UVB | 292,31 |

Les résultats obtenus démontrent qu'en présence de l'actif selon l'invention l'activité de la SOD augmente. L'intensité des bandes, représentative de l'activité de la SOD, double lorsque l'actif est appliqué sur des cellules ayant subi une irradiation aux UVB, par rapport aux cellules n'ayant pas reçu l'actif.

### III. Dosage de l'activité Catalase :

La Catalase est une des enzymes les plus importantes intervenant dans le système de défense contre les radicaux libres. C'est un puissant antioxydant cellulaire. Elle agit en réduisant le peroxyde d'hydrogène (H₂0₂), produit par la SOD, en oxygène et en eau (O₂ et H₂O). L'étude de l'activité de la Catalase s'effectue par des mesures spectrophotométriques. L'activité, présente dans l'échantillon, est calculée en mesurant la vitesse de disparition du peroxyde d'hydrogène.

Lors de leur préparation, les échantillons à tester sont traités avec l'extrait selon l'invention, à 1 %, 24 heures avant l'irradiation, pendant l'irradiation aux UVB à 100 mJ/cm², et encore 30 minutes après l'irradiation. La cinétique de disparition de l'H₂O₂ dans l'échantillon, dilué dans un tampon phosphate, est mesurée à une longueur d'onde de 240 nm ; le zéro aura préalablement été effectué avec un tampon phosphate à 50 mM, puis la concentration du substrat (H₂O₂), dans ce même tampon, aura été ajustée à 14 mM, ce qui correspond à une absorbance comprise entre 0,52 et 0,55.

Cette étude met en évidence une augmentation de l'activité de la catalase lorsque les cellules sont traitées avec l'actif par comparaison avec les cellules non traitées.

### IV. Dosage de la carbonylation des protéines :

La carbonylation des protéines est un phénomène résultant du stress oxydant et de ses conséquences : l'oxydation des macromolécules par les radicaux libres. Cette carbonylation provient du clivage oxydatif des protéines ou d'une oxydation des résidus arginine, lysine, proline ou thréonine. Le dosage de la carbonylation des protéines s'effectue grâce à une technique EIA « Enzyme Immuno Assay ».

La première étape de cette méthode consiste à complexer le DNP avec les échantillons, d'une part, et d'autre part, avec une gamme de BSA oxydée. Ce réactif se fixe spécifiquement sur les groupements carbonyls qui seront dosés par méthode ELISA, grâce à un anticorps anti-DNP couplé à une peroxydase. La gamme de BSA oxydée (dont on connaît la concentration en groupements carbonyls) est utilisée pour l'étalonnage. Pour cette manipulation, les échantillons ont été mis en présence de l'actif selon l'invention à 1 %, 24 heures avant et pendant l'irradiation aux UVB à 100 mJ/cm².

Une fois les échantillons et la gamme complexés avec le réactif DNP, ils sont déposés sur une plaque 96 puits pendant toute une nuit, puis chaque puits est saturé avec de la BSA réduite. Les puits sont, par la suite, lavés et marqués par l'anticorps anti-DNP biotinylé. Un nouveau lavage est effectué afin de coupler le complexe streptavidine-peroxidase avec la biotine. Après un dernier lavage, le substrat de l'enzyme, le TMB, est ajouté dans chaque puits. La lecture est effectuée à 490 nm après addition d'acide sulfurique 2,5 M. La concentration en groupement carbonyl de chaque échantillon est déterminée grâce à la gamme de BSA oxydée.

Le tableau ci-dessous illustre la concentration en groupement carbonyl (en µmol/g de protéines) pour chaque échantillon en fonction des différentes conditions étudiées.

| **Conditions d'étude** | **Concentration en gpt carbonyl (µmol/g de prot.)** |
|---|---|
| - actif / - UVB | 2,31 |
| + actif / - UVB | 1,92 |
| - actif / + UVB | 2,57 |
| + actif / + UVB | 1,90 |

Les résultats obtenus démontrent une diminution de la carbonylation des protéines lorsque les cellules sont traitées avec l'actif selon l'invention. On observe plus particulièrement une importante diminution de cette carbonylation lorsque les cellules sont traitées avec l'actif et soumises à une irradiation, par comparaison aux cellules irradiées mais non traitées avec l'actif.

### V. Conclusion

La SOD et la catalase sont des systèmes enzymatiques spécialisés dans la protection des cellules contre les dommages occasionnés par les radicaux libres. Ainsi, une augmentation de l'activité de la SOD et de la catalase résulte d'une activation du système de protection cellulaire existant naturellement, cette activation permettant une meilleure protection contre les stress oxydants. Ces tests nous permettent donc de démontrer que l'extrait d'une espèce végétale diploïde appartenant au genre Triticum selon l'invention, agissent en tant qu'agent antioxydant et anti-radicalaire. Ces conclusions sont, par ailleurs, confortées par l'évaluation de la quantité de protéine carbonylée, un marqueur des dommages provoqués par les radicaux libres.

### Exemple 3 : Mise en évidence de l'effet de l'extrait selon l'invention sur la viabilité cellulaire.

### I. Test de cytotoxicité par fixation du rouge neutre.

Des fibroblastes primaires sont ensemencés sur une plaque 96 puits (à 20000 cellules par puits environ). Une fois que les cellules ont atteint 70 % de confluence, l'extrait selon l'invention, dilué à 1 %, est appliqué sur la plaque durant 24 heures. Une moitié de la plaque est alors irradiée par des UVB à 100 mJ/cm² et l'autre moitié non irradiée. Les cellules sont ensuite remises en culture pendant 24 h en présence de l'actif, puis incubées en présence de rouge neutre.

Le rouge neutre (un colorant « supravital ») se fixe spécifiquement aux lysosomes des cellules vivantes, la coloration sera ainsi proportionnelle à la viabilité des cellules. Après l'ajout d'une solution de révélation, solubilisant le rouge neutre, l'absorbance est mesurée à 540 nm.

Les résultats obtenus, représentés dans le tableau ci-dessous, représentent le pourcentage de viabilité cellulaire en fonction des différentes conditions étudiées, par rapport à la condition contrôle.

| **Conditions d'étude** | **% de viabilité cellulaire** |
|---|---|
| - actif / - UVB | 100 |

| | |
|---|---|
| + actif / - UVB | 96,70 |
| - actif / + UVB | 80,00 |
| + actif / + UVB | 94,50 |

Ces résultats nous permettent de conclure que l'extrait selon l'invention n'est pas nocif pour les cellules, et bien au contraire, qu'il les protège lorsque celles-ci sont soumises à des agressions d'origines extérieures, notamment les rayonnements UV. On observe, en effet, une augmentation de la viabilité cellulaire de 14,5 % lorsque les cellules sont soumises à une irradiation aux UVB et traitées avec l'actif selon l'invention par comparaison avec les cellules non traitées avec l'actif. Ces résultats démontrent ainsi clairement que l'extrait selon l'invention génère un effet protecteur important au niveau cellulaire.

### II. Visualisation et quantification des lésions de l'ADN.

Une étude de l'effet cytoprotecteur de l'actif selon l'invention, a été effectuée en évaluant l'incidence de l'extrait selon l'invention, sur les dommages provoqués au niveau de l'ADN. Cette étude a été réalisée grâce au test des comètes (ou « Single Cell Gel Electrophoresis »), une technique micro-électrophorétique courte et sensible qui permet de visualiser et de quantifier les cassures de l'ADN sur des cellules individuelles.

Une lignée de fibroblastes primaires est ensemencée sur des boîtes de diamètres 100, une fois les cellules arrivées à 70 % de confluence, les échantillons sont préparés et sont traités avec l'actif, dilué à 1 %, 24 heures avant l'irradiation, pendant l'irradiation UVB à 100 mJ/cm², et 24 heures après l'irradiation. Des boîtes non traitées par l'actif servent de contrôle. Une suspension cellulaire à 100 000 cellules/mL est ensuite réalisée, 500 µL sont prélevés, inclus dans une solution d'agarose et coulés entre lame et lamelle. Les cellules sont lysées à froid. L'ADN est ensuite dénaturé par un tampon alcalin suivi d'une courte électrophorèse (250 mA pendant 30 minutes), et il est mis en évidence par ajout de l'iodure de propidium. Les lames sont alors observées au microscope à fluorescence. L'ADN d'une cellule altérée s'étire vers l'anode proportionnellement au nombre de cassures et forme une comète, l'ADN fortement dégradé se retrouve dans la "queue" de la comète. Une cellule intacte reste ronde, l'ADN restant compacté au niveau de la "tête" de la comète.

L'évaluation des lésions de l'ADN s'effectue à l'aide d'un logiciel analyseur d'image qui permet de déterminer le pourcentage de la dégradation d'ADN, par l'évaluation quantitative du « Tail Moment », un paramètre se définissant comme le produit de la longueur de la comète par le pourcentage d'ADN dans sa partie distale.

Le tableau ci-dessous représente l'évaluation des « Tail Moment » mesurés dans des fibroblastes traités suivant les différentes conditions, ainsi que le pourcentage de ce « Tail moment» par rapport au contrôle UV, c'est-à-dire à la condition où les cellules sont soumises à une irradiation sans que l'actif soit appliqué. Le contrôle UV représentant la condition où le « Tail Moment » est le plus important, c'est-à-dire la condition où l'ADN est le plus endommagé.

| **Conditions d'étude** | **Mesure du « Tail Moment »** | **% du « Tail Moment » par rapport au contrôle UV** |
|---|---|---|
| - actif / - UVB | 2648,51 | 2,99 |
| - actif / + UVB | 88592,41 | 100 |
| + actif / - UVB | 1576,82 | 1,78 |
| + actif / + UVB | 23696,54 | 26,75 |

Les résultats obtenus démontrent que les cellules soumises à une irradiation aux UV subissent d'importants dommages au niveau de l'ADN, tandis que les cellules traitées avec l'extrait selon l'invention en ont beaucoup moins. En effet, ces résultats démontrent que les cellules traitées avec l'actif ont une protection qui augmente de 73 % lorsque les cellules ont subi une irradiation, par rapport aux cellules non traitées. Ces résultats nous permettent de conclure que l'extrait d'une espèce végétale diploïde appartenant au genre Triticum selon l'invention joue un rôle important au niveau de la protection de l'ADN.

### Exemple 4 : Préparation de compositions.

### Les quantités indiquées sont données en pourcentage de poids.

### 1 - Crème de soin anti-rides:

| **Noms commerciaux** | **Noms INCI** | **% massique** |
|---|---|---|
| *PHASE A* | | |
| Montanov 68 | Cetearyl Alcohol (and) Cetearyl Glucoside | 6.00 |
| Squalane | Squalane | 3.00 |
| Cetiol SB 45 | Butyrospermum Parkii (Shea Butter) | 2.00 |
| Waglinol 250 | Cetearyl Ethylhexanoate | 3.00 |
| Amerchol L- 101 | Mineral Oil (and) Lanolin Alcohol | 2.00 |
| Abil 350 | Dimethicone | 1.50 |
| BHT | BHT | 0.01 |

| *PHASE B* | | |
|---|---|---|
| Eau déminéralisée | Aqua (Water) | qsp |
| Butylene Glycol | Butylene Glycol | 2.00 |
| Glucam E10 | Methyl Gluceth-10 | 1.00 |
| Allantoin | Allantoin | 0.15 |
| Carbopol Ultrez 10 | Carbomer | 0.20 |

| *PHASE C* | | |
|---|---|---|
| Huile d'Avocat | Persea Gratissima (Avocado) Oil | 1.25 |
| Phenonip | Phenoxyethanol (and) Methylparaben (and) Ethylparaben (and) Butylparaben (and) Propylparaben (and) Isobutylparaben | 0.75 |

| *PHASE D* | | |
|---|---|---|
| TEA | Triethanolamine | 0.18 |

| *PHASE E* | | |
|---|---|---|
| Extrait selon l'exemple 1 | | 1.00 |
| Parfum | Parfum (Fragrance) | qsp |
| Colorant | | qsp |

Les constituants de la phase A et de la phase B sont chauffés séparément à une température comprise entre 65°C et 70°C, la phase C est incorporée, puis la phase A est émulsionnée dans la phase B. Le Carbomer est neutralisé avec la phase D à une température aux alentours de 45°C. La phase E est ensuite additionnée sous agitation et le refroidissement est poursuivi jusqu'à 25°C.

### 2 - Lait corporel anti-âge:

| ***Noms commerciaux*** | ***Noms INCI*** | **% *massique*** |
|---|---|---|
| *PHASE A* | | |
| Eau déminéralisée | Aqua (Water) | qsp |
| Carbopol EDT 2020 | Acrylates/C10-30 Alkylacrylate | 0.10 |

| ***Noms commerciaux*** | ***Noms INCI*** | **% *massique*** |
|---|---|---|
| | Crosspolymer | |
| Glycerine | Glycerin | 1.20 |
| EDTA | Trisodium EDTA | 0.65 |
| Propylene Glycol | Propylene Glycol | 1.50 |

| *PHASE B* | | |
|---|---|---|
| Miglyol 812 | Caprylic/Capric Triglyceride | 2.50 |
| Amerchol L 101 | Mineral Oil (and) Lanolin Alcohol | 2.00 |
| Squalane | Squalane | 1.50 |
| Cetiol SN | Cetearyl Isononanoate | 2.00 |
| Stearine TP | Stearic Acid | 2.00 |
| Tegin | Glyceryl Stearate SE | 3.00 |
| Lanette 16 | Cetyl Alcohol | 0.20 |
| Dow Corning 200 Fluid | Dimethicone | 0.50 |
| Phenonip | Phenoxyethanol (and) Methylparaben (and) Ethylparaben (and) Butylparaben (and) Propylparaben (and) Isobutylparaben | 0.70 |

| *PHASE C* | | |
|---|---|---|
| TEA | Triethanolamine | 0.08 |

| *PHASE D* | | |
|---|---|---|
| Extrait selon l'exemple 1 | | 1.00 |
| Parfum | Parfum (Fragrance) | qsp |
| Colorant | | qsp |

Les constituants de la phase A et de la phase B sont chauffés séparément à une température comprise entre 70°C et 75°C. La phase B est émulsionnée dans A sous agitation « Staro ». Après un refroidissement jusqu'à 50°C, le mélange est neutralisé avec la phase C. La phase D est ensuite additionnée lorsque la température se situe en dessous de 40°C. Le refroidissement est poursuivi jusqu'à 25°C sous agitation lente.

### 3 - Crème protection solaire:

| ***Noms commerciaux*** | ***Noms INCI*** | ***% massique*** |
|---|---|---|
| *PHASE A* | | |
| ***Noms commerciaux*** | ***Noms INCI*** | ***% massique*** |
| Eau déminéralisée | Aqua (Water) | qsp |
| Pemulen TR1 | Acrylates/C10-30 Alkyl Acrylate Crosspolymer | 0.40 |
| Glycerine | Glycerin | 3.00 |
| Nipastat Sodium | Sodium Methylparaben (and) Sodium Ethylparaben (and) Sodium Butyl paraben (and) Sodium Propylparaben (and) Sodium Isobutylparaben | 0.15 |

| *PHASE B* | | |
|---|---|---|
| Parsol MCX | Ethylhexyl Methoxycinnamate | 7.50 |
| Eusolex 4360 | Benzophenone-3 | 3.00 |
| Parsol 1789 | Butyl Methoxydibenzoylmethane | 2.00 |
| Myritol 318 | Caprylic/Capric Triglyceride | 4.00 |
| Emulgade SEV | Hydrogenated Palm Glycerides (and) Ceteareth-20 (and) Ceteareth-12 (and) Cetearyl Alcohol | 5.00 |
| Propylparaben | Propylparaben | 0.15 |
| Nacol 16-98 | Cetyl Alcohol | 1.00 |

| *PHASE C* | | |
|---|---|---|
| TEA | Triethanolamine | 0.20 |

| *PHASE D* | | |
|---|---|---|
| Extrait selon l'exemple 1 | | 0.50 |
| Parfum | Parfum (Fragrance) | qsp |
| Colorant | | qsp |

Les constituants de la phase A et de la phase B sont chauffés séparément à une température comprise entre 70°C et 75°C. La phase B est émulsionnée dans A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

## Revendications

1. Composition comprenant une quantité efficace d'un extrait aqueux de graines de *Triticum monococcum,* dans un milieu cosmétiquement acceptable, destinée à être utilisée comme agent photo-protecteur dans le traitement de la peau contre des agressions dues aux rayonnements UV.

2. Composition destinée à être utilisée selon la revendication 1, **caractérisée en ce que** ledit extrait est réalisé à partir de farines issues des graines de *Triticum monococcum.*

3. Composition destinée à être utilisée selon l'une des revendications 1 ou 2, pour lutter contre et/ou prévenir le vieillissement photo-induit.

4. Composition comprenant une quantité efficace d'un extrait aqueux de graines de *Triticum monococcum,* dans un milieu cosmétiquement acceptable, seul ou en association avec au moins un autre agent actif, destinée à être utilisée en tant qu'agent antioxydant et/ou anti-radicalaire, dans le traitement du stress oxydatif de la peau.

5. Composition comprenant une quantité efficace d'un extrait aqueux de graines de *Triticum monococcum,* dans un milieu cosmétiquement acceptable, destinée à être utilisée dans le traitement de la peau et les phanères contre tous les types d'agressions extérieures.

6. Composition destinée à être utilisée selon la revendication 5, **caractérisée en ce que** les agressions extérieures sont choisies parmi la pollution, les produits à caractère irritant tels que les tensioactifs, les conservateurs ou les parfums.

7. Composition destinée à être utilisée selon l'une quelconque des revendications précédentes, **caractérisée en ce que** l'extrait est présent dans la composition le contenant à une quantité comprise entre 0,0001 % et 10 % en poids, préférentiellement à une quantité comprise entre 0,001 % et 5 % en poids, et d'une manière encore plus préférentielle à une quantité comprise entre 0,05 % et 3 % en poids par rapport au poids total de la composition finale.

8. Composition destinée à être utiliséeselon l'une quelconque des revendications précédentes, **caractérisée en ce que** l'extrait est préalablement solubilisé dans un ou plusieurs solvants cosmétiquement acceptables comme l'eau, l'éthanol, le propylène glycol, le butylène glycol, le dipropylène glycol, les diglycols éthoxylés ou propoxylés, les polyols cycliques, la vaseline, une huile végétale ou tout mélange de ces solvants.

9. Composition destinée à être utilisée selon l'une quelconque des revendications 1 à 7, **caractérisée en ce que** l'extrait est préalablement solubilisé dans, ou fixé sur, tout vecteur cosmétiquement acceptable.

10. Composition destinée à être utilisée selon l'une quelconque des revendications précédentes, **caractérisée en ce qu'**elle est à usage topique.

11. Composition destinée à être utilisée selon la revendication 10, **caractérisée en ce qu'**elle se présente sous forme d'une solution aqueuse, hydroalcoolique ou huileuse ou sous la forme d'une émulsion huile-dans-eau, eau-dans-huile ou émulsions multiples ou sous forme de crèmes, de suspensions, ou encore poudres, ces compositions étant fluides ou solides et ayant l'aspect d'une crème, d'une lotion, d'un lait, d'un sérum, d'une pommade, d'un gel, d'une pâte, d'une mousse ou d'un stick.

12. Composition destinée à être utilisée selon la revendication 11, comprenant en outre au moins un adjuvant choisi parmi les solvants, les épaississants, les diluants, les antioxydants, les colorants, les filtres solaires, les agents auto-bronzants, les pigments, les charges, les conservateurs, les parfums, les absorbeurs d'odeur, les actifs cosmétiques, les huiles essentielles, les vitamines, les acides gras essentiels, les tensioactifs ou les polymères filmogènes.

13. Composition dermatologique, comprenant dans un milieu pharmaceutiquement acceptable, au moins une quantité efficace d'un extrait aqueux de graines de *Triticum monococcum,* destinée à être utilisée dans le traitement de l'inflammation cutanée.

14. Composition destinée à être utilisée selon la revendication 13, **caractérisée en ce que** ledit extrait est réalisé à partir de farines issues des graines de *Triticum monococcum.*

## Patentansprüche

1. Zusammensetzung, umfassend eine wirksame Menge eines wässrigen Extrakts von Samen von *Triticum monococcum* in einem kosmetisch annehmbaren Medium, ausgelegt, um als Lichtschutzmittel bei der Behandlung der Haut gegen Aggressionen aufgrund von UV-Strahlungen verwendet zu werden.

2. Zusammensetzung, ausgelegt, um gemäß Anspruch 1 verwendet zu werden, **dadurch gekennzeichnet, dass** der Extrakt auf der Grundlage von Mehlen aus den Samen von *Triticum monococcum* hergestellt ist.

3. Zusammensetzung, ausgelegt, um gemäß einem der Ansprüche 1 oder 2 verwendet zu werden, um gegen die photoinduzierte Alterung zu kämpfen und/oder dieser vorzubeugen.

4. Zusammensetzung, umfassend eine wirksame Menge eines wässrigen Extrakts von Samen von *Triticum monococcum* in einem kosmetisch annehmbaren Medium, alleine oder in Verbindung mit mindestens einem anderen Wirkstoff, ausgelegt, um als Antioxidationsmittel und/oder Antiradikal bei der Behandlung des oxidativen Stresses der Haut verwendet zu werden.

5. Zusammensetzung, umfassend eine wirksame Menge eines wässrigen Extrakts von Samen von *Triticum monococcum* in einem kosmetisch annehmbaren Medium, ausgelegt, um bei der Behandlung der Haut und der Hautanhangsgebilde gegen alle Arten von äußeren Aggressionen verwendet zu werden.

6. Zusammensetzung, ausgelegt, um nach Anspruch 5 verwendet zu werden, **dadurch gekennzeichnet, dass** die äußeren Aggressionen ausgewählt sind aus Verschmutzung, Produkten irritierender Art wie z.B. oberflächenaktiven Stoffen, Konservierungsstoffen und Parfums.

7. Zusammensetzung, ausgelegt, um nach einem der vorhergehenden Ansprüche verwendet zu werden, **dadurch gekennzeichnet, dass** der Extrakt in der Zusammensetzung, die es enthält, in einer Menge, die zwischen 0,0001 Gew.% und 10 Gew.% liegt, vorzugsweise in einer Menge, die zwischen 0,001 Gew.% und 5 Gew.% liegt, und noch bevorzugter in einer Menge, die zwischen 0,05 Gew.% und 3 Gew.% liegt, mit Bezug auf das Gesamtgewicht der endgültigen Zusammensetzung vorhanden ist.

8. Zusammensetzung, ausgelegt, um nach einem der vorhergehenden Ansprüche verwendet zu werden, **dadurch gekennzeichnet, dass** der Extrakt zuvor in einem oder in mehreren Lösemitteln solubilisiert wird, die kosmetisch akzeptabel sind, wie z.B. Wasser, Ethanol, Propylenglycol, Butylenglycol, Dipropylenglycol, ethoxylierten oder propoxylierten Diglykolen, cyclischen Polyolen, Vaselin, einem pflanzlichen Öl oder jeder Mischung dieser Lösemittel.

9. Zusammensetzung, ausgelegt, um nach einem der Ansprüche 1 - 7 verwendet zu werden, **dadurch gekennzeichnet, dass** der Extrakt zuvor in jedem kosmetisch annehmbaren Vektor solubilisiert oder darauf fixiert wird.

10. Zusammensetzung, ausgelegt, um nach einem der vorhergehenden Ansprüche verwendet zu werden, **dadurch gekennzeichnet, dass** sie zur topischen Verwendung ist.

11. Zusammensetzung, ausgelegt, um nach Anspruch 10 verwendet zu werden, **dadurch gekennzeichnet, dass** sie die Form einer wässrigen, hydroalkoholischen oder ölhaltigen oder die Form einer Öl-in-Wasser-, Wasser-in-Öl-Emulsion oder multipler Emulsionen oder die Form von Cremes, Suspensionen oder auch Pulvern aufweist, wobei diese Zusammensetzungen flüssig oder fest sind und das Aussehen einer Creme, einer Lotion, einer Milch, eines Serums, einer Salbe, eines Gels, einer Paste, eines Schaums oder eines Sticks aufweisen.

12. Zusammensetzung, ausgelegt, um nach Anspruch 11 verwendet zu werden, umfassend außerdem mindestens ein Hilfsmittel, ausgewählt aus den Lösemitteln, den Verdickungsmitteln, den Verdünnungsmitteln, den Antioxidationsmitteln, den Farbstoffen, den Sonnenfiltern, den Selbstbräunungsmitteln, den Pigmenten, den Chargen, den Konservierungsstoffen, den Parfums, den Geruchsabsorbern, den kosmetisch aktiven Stoffen, den essenziellen Ölen, den Vitaminen, den essenziellen Fettsäuren, den oberflächenaktiven Stoffen oder den filmbildenden Polymeren.

13. Dermatologische Zusammensetzung, umfassend in einem pharmazeutisch annehmbaren Milieu mindestens eine wirksame Menge eines wässrigen Extrakts von Samen von *Triticum monococcum,* ausgelegt, um bei der Behandlung der Hautentzündung verwendet zu werden.

14. Zusammensetzung, ausgelegt, um nach Anspruch 13 verwendet zu werden, **dadurch gekennzeichnet, dass** der Extrakt auf der Grundlage von Mehlen aus den Samen von *Triticum monococcum* hergestellt ist.

## Claims

1. Composition comprising an effective quantity of an aqueous extract of *Triticum monococcum* grains, in a cosmetically acceptable medium, intended to be used as a photoprotective agent in the treatment of the skin against attacks due to UV radiation.

2. Composition intended to be used according to claim 1, **characterised in that** said extract is produced from flour issuing from *Triticum monococcum* grains.

3. Composition intended to be used according to one of claims 1 or 2, for combatting and/or preventing photoinduced ageing.

4. Composition comprising an effective quantity of an aqueous extract of *Triticum monococcum* grains, in a cosmetically acceptable medium, alone or in association with at least one other active agent, intended to be used as an antioxidant and/or antiradical agent, in the treatment of oxidative stress of the skin.

5. Composition comprising an effective quantity of an aqueous extract of *Triticum monococcum* grains, in a cosmetically acceptable medium, intended to be used in the treatment of skin and the keratinous appendages against all types of external attacks.

6. Composition intended to be used according to claim 5, **characterised in that** the external attacks are chosen from pollution and substances of an irritating character such as surfactants, preservatives or perfumes.

7. Composition intended to be used according to any one of the preceding claims, **characterised in that** the extract is present in the composition containing it at a quantity of between 0.0001% and 10% by weight, preferentially at a quantity of between 0.001% and 5% by weight, and even more preferentially at a quantity of between 0.05% and 3% by weight with respect to the total weight of the final composition.

8. Composition intended to be used according to any one of the preceding claims, **characterised in that** the extract is previously solubilised in one or more cosmetically acceptable solvents such as water, ethanol, propylene glycol, butylene glycol, dipropylene glycol, ethoxylated or propoxylated diglycols, cyclic polyols, Vaseline, a plant oil or any mixture of these solvents.

9. Composition intended to be used according to any one of claims 1 to 7, **characterised in that** the extract is previously solubilised in, or fixed on, any cosmetically acceptable carrier.

10. Composition intended to be used according to any one of the preceding claims, **characterised in that** it is for topical use.

11. Composition intended to be used according to claim 10, **characterised in that** it is in the form of an aqueous, hydroalcoholic or oily solution or in the form of an oil-in-water or water-in-oil emulsion or multiple emulsions or in the form of creams, suspensions or powders, these compositions being fluid or solid and having the appearance of a cream, a lotion, a milk, a serum, an ointment, a gel, a paste, a foam or a stick.

12. Composition intended to be used according to claim 11, further comprising at least one adjuvant chosen from solvents, thickeners, diluents, antioxidants, colorants, sun filters, self-tanning agents, pigments, fillers, preservatives, perfumes, odour absorbers, cosmetic active agents, essential oils, vitamins, essential fatty acids, surfactants or film-forming polymers.

13. Dermatological composition, comprising, in a pharmaceutically acceptable medium, at least an effective quantity of an aqueous extract of *Triticum monococcum* grains, intended to be used in the treatment of skin inflammation.

14. Composition intended to be used according to claim 13, **characterised in that** said extract is produced from flours issuing from *Triticum monococcum* grains.
